# FASCICULE DE BREVET EUROPEEN

(11) **EP 0 682 006 B1**
(45) Date de publication et mention de la délivrance du brevet: **07.04.1999**
(21) Numéro de dépôt: 94107202.7
(22) Date de dépôt: 09.05.1994
(51) Int. Cl.: C07C 67/60, C07C 69/587

(54) **Procédé de préparation d'un concentrat d'esters d'acides gras polyinsaturés**
Verfahren zur Herstellung eines Konzentrates von Estern von mehrfach ungesättigten Fettsäuren
Process for the preparation of a concentrate of esters of polyunsaturated fatty acids

(43) Date de publication de la demande: 15.11.1995
(73) Titulaire: SOCIETE DES PRODUITS NESTLE S.A., 1800 Vevey (CH)
(72) Inventeur: Bertoli, Constantin, CH-1032 Romanel S/Lausanne (CH); Fumeaux, René, CH-1814 La Tour-De-Peilz (CH); Grand-Guillaume Perrenoud, Marie-Claude, CH-1618 Chatel-St-Denis (CH); Wang, Junkuan, CH-1010 Lausanne (CH)
(74) Mandataire: Archambault, Jean

(56) Documents cités:
- EP-A- 0 271 747
- FR-A- 1 603 383
- US-A- 4 377 526

## Description

L'invention concerne un procédé de préparation d'un concentrat d'acides gras polyinsaturés sous forme d'esters éthyliques, notamment des acides gras essentiels des familles n-3 et n-6, en particulier les acides gammalinolénique (GLA), linoléique (LA), éicosapentaénoique (EPA) et docosahexaénoique (DHA).

Dans EP-B-178442 ou US-A-4776984, on prépare un concentrat enrichi en acides gras de la famille n-6, en particulier en GLA à partir d'un mélange d'acides gras obtenu par saponification d'une huile de pépins contenant le GLA, par exemple l'huile de pépins des cassis. Le procédé consiste en la saponification de l'huile, le fractionnement des acides gras par complexation avec l'urée en présence de méthanol et l'extraction de la fraction enrichie en GLA avec utilisation d'acide chlorhydrique. Ce procédé permet d'obtenir une teneur d'environ 77 à 81% de GLA dans le mélange d'acides gras final.

Lorsque l'on désire encore enrichir le mélange précédent, de manière à obtenir, par exemple, une teneur en acide gras polyinsaturé d'au moins 90% en poids, on a recours d'ordinaire à la séparation chromatographique des acides gras.

L'application à l'échelle industrielle du procédé connu présente des inconvénients: le méthanol provoque une contamination du produit final par formation d'esters méthyliques d'acides gras toxiques. De plus, l'utilisation d'acide chlorhydrique corrode les installations.

US-A-4377526 Concerne un procédé de raffinage d'huile de poisson utilisant la complexation sélective des acides gras ou de leurs esters avec l'urée comme l'une des étapes du raffinage. Nous remarquons que l'antériorité ne fait aucune distinction entre le traitement des acides gras et celui des esters, ce qui laisse supposer que ces entités sont équivalentes du point de vue du procédé: les exemples 1, 3 et 4 traitent les esters éthyliques et les exemples 2 et 5 les acides. Quelque soit la matière première mise en oeuvre, le procédé comprend plusieurs étapes essentielles: la séparation par distillation fractionnée et la purification sur colonne de silice du mélange à traiter par l'urée, ou du substrat après réaction avec l'urée.

FR-A-1603383 A trait à un procédé de raffinage d'un mélange spécifique d'esters méthyliques ou éthyliques d'acides gras obtenu par interestérification aléatoire d'huile de bourrache, comprenant la complexation du dit mélange avec l'urée, séparation de la fraction liquide, extraction de la fraction liquide avec l'éther éthylique et élution d'une fraction enrichie en acide gammalinolénique par passage sur une colonne de résine d'échange d'ions chargée de nitrate d'argent.

Le procédé selon l'invention est caractérisé par le fait que l'on conduit l'éthanolyse d'une huile riche en acides gras polyinsaturés préalablement raffinée, avec l'éthanol en présence d'un catalyseur pour obtenir des esters éthyliques d'acides gras, que l'on complexe ces esters avec l'urée en solution dans l'éthanol de manière à former un complexe d'inclusion insoluble, que l'on sépare le complexe d'inclusion et que l'on recueille une fraction d'esters éthyliques d'acides gras enrichie en acides gras polyinsaturés dans la phase liquide, que l'on élimine partiellement l'éthanol de la phase liquide, puis que l'on extrait le concentrat avec un solvant, que l'on traite le concentrat en solution avec 10 à 40 % en poids de charbon actif par rapport au concentrat brut, à la température ambiante, que l'on sépare le charbon actif et que l'on élimine le solvant par évaporation sous vide .

Dans le contexte de l'invention, on entend par "huile riche en acides gras polyinsaturés" une huile végétale riche en GLA, par exemple l'huile d'onagre, l'huile de bourrache ou une huile de pépins de fruits du genre Ribes, en particulier de cassis, une huile végétale riche en acide linoléique (LA) et pauvre en acide alphalinolénique (ALA), par exemple l'huile de passiflore ou l'huile de maïs ou encore une huile d'animaux marins contenant les acides EPA et DHA.

On entend par "raffinage préalable" une série de traitements effectués de manière classique après extraction de l'huile, par exemple par pression et extraction par solvant, par exemple à l'hexane, du gâteau de pressage, suivie d'une évaporation de l'hexane. Ces traitements de raffinage comprennent un dégommage, par exemple au silicagel, une neutralisation par une base, une décoloration sous vide en présence de terre décolorante, une désodorisation par entrainement à la vapeur et une stabilisation par addition d'antioxydant liposolubilisé.

Selon l'invention, on fait subir à l'huile raffinée une éthanolyse, c'est à dire que l'on prépare les esters éthyliques des acides gras en déplaçant le glycérol des acyl-glycérols avec l'éthanol en présence d'une quantité appropriée de catalyseur, par exemple l'hydroxyde de sodium ou de potassium ou l'éthylate de sodium. On conduit l'éthanolyse en mélangeant l'huile raffinée avec, en poids, de 1,5 à 3 équivalents, de préférence avec environ 2 équivalents d'éthanol anhydre dans lequel on a dissout de 0,5 à 1 %, de préférence 0,8 % de catalyseur, basé sur la charge d'huile. On peut effectuer la réaction à une température de 30 à 80°C, de préférence d'environ 50°C pendant 10 à 60 min. et sous agitation. Après repos pendant environ 1h, on observe la présence de deux phases liquides, une légère contenant les esters éthyliques et l'autre lourde contenant le glycérol et on sépare le glycérol formé par centrifugation ou décantation.

Cette étape est suivie du fractionnement des esters éthyliques par complexation sélective des esters éthyliques d'acides gras, principalemenrt saturés et mono-insaturés avec l'urée en présence d'éthanol, esters qui forment des complexes d'inclusion insolubles avec l'urée. Des esters éthyliques d'acides gras de degré d'insaturation plus élevés peuvent également former des complexes insolubles avec l'urée selon la température de refroidissement, la quantité d'urée, la position et le nombre des doubles liaisons. La phase liquide est enrichie en les esters d'acides gras polyinsaturés désirés. La quantité d'urée mise en oeuvre est proportionnelle à la quantité d'esters éthyliques d'acides gras désirés dans la phase liquide. On utilise ainsi un rapport pondéral esters éthyliques:urée de 1:2 à 1:4,5, de préférence environ 1:3,2. La quantité d'éthanol mise en oeuvre est avantageusement, en poids, de 6,5 à 15 fois et de préférence environ 9,75 fois la quantité de matière première engagée.

Pour réaliser ce fractionnement, on chauffe le mélange esters éthyliques, urée et éthanol à environ 80°C sous agitation jusqu'à dissolution de l'urée.

Lorsque l'on désire enrichir le mélange dans les esters éthyliques des acides gras polyinsaturés GLA, EPA et DHA, par exemple, on refroidit ensuite la solution à 0-20°C et de préférence à 15-18°C.

Dans le cas d'un enrichissement en LA, par exemple, on refroidit la solution à une température plus élevée que précédemment, par exemple d'environ 40°C.

Une phase solide apparaît, que l'on sépare par centrifugation ou filtration. On recueille la phase liquide dont on élimine partiellement l'éthanol, puis on en extrait les esters éthyliques enrichis en acides gras polyinsaturés avec un solvant, de préférence le n-hexane (ci-après hexane). On réalise cette extraction de préférence en présence d'acide, par exemple l'acide phosphorique aqueux. On obtient de cette manière un concentrat brut d'esters éthyliques d'acides gras polyinsaturés.

La phase solide contient encore une quantité appréciable des esters éthyliques désirés qu'il est souhaitable de récupérer. Pour ce faire, on peut extraire ces esters du complexe solide au moyen d'un solvant, par exemple l'hexane. L'hexane peut ensuite être éliminé, par exemple par évaporation et les esters éthyliques ainsi récupérés être mélangés à la charge destinée au fractionnement ou encore subir un fractionnement séparé dans les conditions usuelles afin d'augmenter le rendement. Les esters résiduels se trouvant dans la phase solide peuvent être entièrement récupérés en ajoutant de l'eau, un acide minéral et de l'hexane. Si ce dernier mélange est utilisé comme matière première du fractionnement, deux fractionnements successifs sont nécessaires, par exemple pour obtenir un concentrat contenant plus de 80% d'ester éthylique de GLA.

Le concentrat brut ainsi préparé doit être raffiné. Dans ce but, on traite le concentrat en solution, de préférence à environ 20% dans l'hexane avec 10 à 40% et de préférence environ 20% en poids de charbon actif (par rapport au concentrat brut), à la température ambiante. Après séparation du charbon actif, par exemple par filtration, on élimine l'hexane, de préférence par évaporation sous vide, ce qui conduit à environ 12% en poids de concentrat d'esters éthyliques d'acides gras polyinsaturés par rapport à l'huile engagée.

Le concentrat ainsi raffiné peut être stabilisé contre l'oxydation en ajoutant, par exemple du DL-alpha-tocophérol et du palmitate d'ascorbyle dissous dans l'éthanol. Après mélange, on élimine l'éthanol et les traces d'hexane restant du concentrat en le purgeant avec de l'azote, par exemple à environ 40°C et sous une pression absolue d'environ 300 mbar.

Selon une variante préférée du procédé conduisant à un concentrat raffiné final d'esters éthyliques d'acides gras contenant une quantité extrêmement faible, voire négligeable d'acides gras libres et, par conséquent une teneur en esters d'acides gras polyinsaturés désirés plus élevée, on procède à une évaporation de l'éthanol juste après l'éthanolyse, puis après séparation du glycérol, on procède au préraffinage de l'éthanolysat avec du charbon actif en présence d'un solvant, de préférence l'hexane, puis on élimine le solvant, les autres étapes étant inchangées.

Le concentrat d'esters éthyliques d'acides gras polyinsaturés obtenu par le procédé de l'invention peut être utilisé dans les applications habituelles des acides gras polyinsaturés, notamment dans des compositions nutritives, pharmaceutiques, cosmétiques et dermatologiques, telles que décrites, par exemple dans les brevets EP-B-092085 et EP-B-092076.

Les exemples ci-après illustrent l'invention. Dans ceux-ci, les pourcentages et parties sont en poids, sauf indication contraire.

### Exemple 1

On procède à l'extraction par pressage mécanique à froid de graines de bourrache (Borago officinalis), puis extraction de l'huile du gâteau de pressage à l'hexane, suivie d'une évaporation de l'hexane. On raffine alors l'huile brute par dégommage au silicagel amorphe, neutralisation par une solution aqueuse d'hydroxyde de sodium, blanchiment par contact avec une terre décolorante activée en présence de silicagel amorphe à 80°C sous vide de 2 mbar, déodorisation par entrainement à la vapeur à 180°C, 3 h sous vide, puis stabilisation contre l'oxydation par addition de palmitate d'ascorbyle. L'huile de bourrache a la composition approximative suivante indiquée dans le tableau 1 ci-après:

**Tableau 1**

| Acides gras | % des acides gras |
|---|---|
| C16:0 | 9-15 |
| C16:1 | <0,4 |
| C18:0 | 3-7 |
| C18:1, delta 9 | 15-19 |
| C18:2, delta 9,12 | 32-38 |
| C18:3, delta 6,9,12 (GLA) | 18-25 |
| C18:3, delta 9,12,15 (ALA) | <1 |
| C20:0 | <0,4 |
| C20:1, delta 9 | 2-4 |
| C22:1, delta 9 | 2-4 |

A 16 kg d'huile de bourrache raffinée, on ajoute 5,12 kg d'une solution à 1,5 d'hydroxyde de sodium dans l'ethanol anhydre (préparée par traitement au reflux pendant 30 min.) dans un réacteur muni de moyens d'agitation. On agite le mélange résultant à 50°C pendant 30 min.. Après 60 min. au repos, deux phases liquides se sont séparées. On élimine le glycérol formé au fond du réacteur constituant la phase liquide lourde par décantation et on recueille la phase légère.

On chauffe un mélange composé de 15,5 kg des esters éthyliques d'acides gras constituant la phase légère précédente, 154 kg d'éthanol technique et 49,6 kg d'urée à 80°C, sous agitation jusqu'à l'apparition d'une solution claire. On refroidit ensuite le mélange à 15°C et il se forme une phase solide. On sépare la phase solide par filtration et on recueille 149,5 kg de phase liquide claire. Après élimination de 80 kg d'éthanol par évaporation sous vide, on ajoute au condensat 80 kg d'eau, 40 g d'une solution aqueuse à 80% d'acide phosphorique et 14 kg d'hexane. Après agitation pendant 10 min., on laisse le mélange au repos pendant 60 min., ce qui produit deux phases. On recueuille la phase supérieure contenant 15% d'esters éthyliques d'acides gras par décantation.

On ajoute 0,4 kg de charbon actif à la solution des esters éthyliques d'acide gras dans l'hexane. Après agitation pendant 60 min. à la température ambiante, on sépare le charbon actif par filtration et on élimine l'hexane par évaporation sous vide. On obtient ainsi 1,8 kg de concentrat d'esters éthyliques d'acides gras (ce qui correspond à un rendement de 12% par rapport à l'huile de départ). Le concentrat a la composition indiquée dans le tableau 2 ci-après, déterminée par chromatographie en phase gazeuse:

**Tableau 2**

| Esters éthyliques d'acides gras (EE) | % |
|---|---|
| C18:2, delta 9,12 | 2 |
| C18:3, delta 6,9,12 (GLA) | 95,9 |
| C18:3, delta 9,12,15 (ALA) | traces |
| C18:4, delta 6,9,12,15 | 0,8 |
| Autres | 1,3 |

On stabilise enfin le concentrat raffiné contre l'oxydation en ajoutant à 1,8 kg de concentrat 900 mg de DL-alpha-tocophérol et 360 mg de palmitate d'ascorbyle dissous dans l'éthanol. Après mélange, on élimine l'éthanol et les traces restantes d'hexane du concentrat en le purgeant à l'azote à 40°C sous 300 mbar absolu.

### Exemple 2

On procède comme à l'exemple 1 au fractionnement des esters d'acides gras de l'huile de pépins de cassis. La composition du concentrat obtenu est indiquée au tableau 3 ci-après:

**Tableau 3**

| %GLA dans l'huile de départ | %GLA-EE dans la fraction enrichie | Rendement (%, par rapport au GLA) |
|---|---|---|
| 15,7 | 74,4 | 56,5 |

### Exemple 3

On applique le procédé de l'exemple 1 au fractionnement des esters éthyliques d'acides gras de l'huile de poisson. L'huile de poisson contient des acides gras polyinsaturés à très longue chaîne de la famille n-3, principalement EPA et DHA.

On détermine la composition (%) des esters d'acides gras du concentrat obtenu par chromatographie en phase gazeuse. Celle-ci est indiquée dans le tableau 4 ci-après:

**Tableau 4**

| Composition des EE (%) | | | | |
|---|---|---|---|---|
| | C18:4 | C20:5 | C22:6 | Autres |
| Huile de poisson | 3,6 | 19,7 | 12,5 | 64,2 |
| | | | | |
| Fraction enrichie sous forme d'ester éthylique | 8 | 49 | 27 | 16 |
| | | | | |
| Rendement (% basé sur la quantité initiale de l'acide gras respectif) | 47 | 52,6 | 45,7 | |

### Exemple 4

On procède à l'enrichissement sélectif de l'huile de maïs en ester éthylique de LA de la même manière que dans l'exemple 1, sauf que le rapport esters éthylique:urée est 1:2,7 au lieu de 1:3,2 et que le mélange fractionné est refroidi à 40°C au lieu de 15°C. Le contenu initial en LA dans l'huile de maïs est 52,3% et le concentrat obtenu contient 92,4% LA-EE, ce qui correspond à un rendement de 59% par rapport à la quantité initiale de LA.

### Exemple 5

On ajoute à 18 kg d'huile de bourrache raffinée, 7,4 kg d'une solution éthanolique à 1,95% d'hydroxyde de sodium, préparée au préalable en agitant l'éthanol et l'hydroxyde de sodium pendant 60 min. à 50-60°C. On agite ensuite le mélange résultant à 50°C pendant 60 min., puis on évapore l'excès d'éthanol à 50°C sous une pression de 100 mbar absolu. Après avoir laissé le mélange au repos pendant 60 min., on sépare soigneusement la phase lourde contenant le glycérol qui s'est déposée au fond du réacteur.

On dissout la phase légère obtenue précédemment, qui représente 17,5 kg de mélange d'esters éthyliques, dans 35 kg d'hexane et on ajoute à la solution 3,5 kg de charbon actif sous agitation à la température ambiante pendant 10 min.. Après avoir éliminé le charbon actif épuisé par filtration, puis évaporé l'hexane, on obtient 16,5 kg d'esters éthyliques d'acides gras préraffinés.

On chauffe un mélange de 161 kg d'éthanol technique et de 53 kg d'urée à 75°C jusqu'à dissolution totale de l'urée. On ajoute alors à la solution éthanolique 16,5 kg des esters éthyliques préraffinés précédents, puis on refroidit ce mélange à 17°C. Il se forme une phase solide que l'on sépare par filtration et 160 kg d'une phase liquide claire que l'on recueille. Après élimination de 100 kg d'éthanol par évaporation sous vide, on ajoute au mélange ainsi concentré 60 kg d'eau, 0,18 kg d'une solution aqueuse d'acide phosphorique à 85% et 12 kg d'hexane. Après agitation pendant 10 min., on laisse le mélange au repos pendant 60 min., puis on recueille par décantation 15,2 kg de phase légère constituant l'éthanolysat et contenant 14,8% d'esters éthyliques d'acides gras.

A ce stade, on effectue un préraffinage de l'éthanolysat avec 0,66 kg de charbon actif. Après agitation pendant 60 min. à la température ambiante, on élimine le charbon actif épuisé par filtration et on élimine l'hexane par évaporation sous vide. On obtient ainsi 2,19 kg de concentrat de GLA-EE.

On ajoute aux 2,19 kg du concentrat précédents 1,06 g de DL-alpha-tocophérol et 0,42 g de palmitate d'ascorbyle dissous dans 6g d'éthanol. Après avoir suffisamment mélangé, on élimine l'éthanol et les traces d'hexane résiduel en purgeant le concentrat avec de l'azote à 40°C et sous une pression de 300 mbar absolu pendant 4 h.

Le concentrat de GLA-EE obtenu contient plus de 97% d'esters éthyliques d'acides gras et moins de 0,2% d'acides gras libres. Le concentrat a la composition indiquée dans le tableau 5 ci-après, déterminée par chromatographie en phase gazeuse:

**Tableau 5**

| Esters éthyliques d'acides gras (EE) | % |
|---|---|
| C18:2, delta 9,12 | 4,4 |
| C18:3, delta 6,9,12 (GLA) | 93,2 |
| C18:3, delta 9,12,15 (ALA) | traces |
| C18:4, delta 6,9,12,15 | 0,2 |
| Autres | 2,2 |

## Revendications

1. Procédé de préparation d'un concentrat d'esters éthyliques d'acides gras polyinsaturés,
caractérisé par le fait que l'on conduit l'éthanolyse d'une huile riche en acides gras polyinsaturés préalablement raffinée, avec l'éthanol en présence d'un catalyseur pour obtenir des esters éthyliques d'acides gras, que l'on complexe ces esters avec l'urée en solution dans l'éthanol de manière à former un complexe d'inclusion insoluble, que l'on sépare le complexe d'inclusion et que l'on recueille une fraction d'esters éthyliques d'acides gras enrichie en acides gras polyinsaturés dans la phase liquide, que l'on élimine partiellement l'éthanol de la phase liquide, puis que l'on en extrait le concentrat avec un solvant, que l'on traite le concentrat en solution avec 10 à 40% en poids de charbon actif par rapport au concentrat brut, à la température ambiante, que l'on sépare le charbon actif et que l'on élimine l'hexane par évaporation sous vide.

2. Procédé selon la revendication 1, caractérisé par le fait que l'on traite une huile végétale riche en acide gammalinolénique choisie parmi l'huile d'onagre, l'huile de bourrache ou une huile de pépins de fruits du genre Ribes, notamment de cassis, une huile végétale riche en acide linoléique et pauvre en acide alphalinolénique, notamment l'huile de passiflore ou l'huile de maïs ou encore une huile d'animaux marins riche en les acides éicosapentaénoique et docosahexaénoique.

3. Procédé selon la revendication 2, contenant l'ester éthylique de l'acide gammalinolénique, caractérisé par le fait que l'on traite l'huile de bourrache.

4. Procédé de préparation d'un concentrat d'esters éthyliques d'acides gras polyinsaturés dans lequel on conduit l'éthanolyse d'une huile riche en acides gras polyinsaturés, caractérisé par le fait que l'on procède à une évaporation de l'éthanol juste après l'éthanolyse, puis après séparation du glycérol, on procède au préraffinage de l'éthanolysat avec du charbon actif en présence d'un solvant, notamment l'hexane, à la température ambiante, que l'on sépare le charbon actif et que l'on élimine l'hexane.

5. Procédé selon la revendication 1 ou 2, caractérisé par le fait que l'on stabilise le concentrat raffiné contre l'oxydation en ajoutant un antioxydant liposolubilisé.

6. Concentrat d'esters éthyliques d'acides gras polyinsaturés obtenu par le procédé selon la revendication 3 à partir de l'huile de bourrache, caractérisé par le fait qu'il contient en poids plus de 97% d'esters éthyliques d'acides gras et moins de 0,2% d'acides gras libres.

7. Utilisation d'un concentrat selon la revendication 6, dans une composition nutritive, cosmétique ou pharmaceutique.

## Patentansprüche

1. Verfahren zur Herstellung eines Konzentrats von Ethylestern von mehrfach ungesättigten Fettsäuren, dadurch gekennzeichnet, daß man die Ethanolyse eines vorher raffinierten Öls, das reich an mehrfach ungesättigten Fettsäuren ist, mit Ethanol in Gegenwart eines Katalysators durchführt, um die Ethylester der Fettsäuren zu erhalten, daß man diese Ester mit Harnstoff in einer Lösung in Ethanol auf eine solche Weise komplexiert, daß ein unlöslicher Einschlußkomplex gebildet wird, daß man den Einschlußkomplex abtrennt und daß man eine Fraktion von Ethylestern von Fettsäuren, die an mehrfach ungesättigten Fettsäuren angereichert sind, in der flüssigen Phase gewinnt, daß man das Ethanol aus der flüssigen Phase teilweise entfernt, daß man das Konzentrat mit einem Lösungsmittel extrahiert, daß man das Konzentrat in Lösung mit 10 bis 40 Gew.-% Aktivkohle, bezogen auf das rohe Konzentrat, bei Umgebungstemperatur behandelt, daß man die Aktivkohle abtrennt und daß man das Lösungsmittel durch Verdampfung bei Unterdruck abtrennt.

2. Verfahren nach Anspruch 1, dadurch gekennzeichnet, daß man ein Pflanzenöl, das reich an gamma-Linolensäure ist und das ausgewählt ist aus Nachtkerzenöl, Borretschöl und einem Öl aus Kernen von Früchten der Gattung Ribes, insbesondere schwarzen Johannisbeeren, ein Pflanzenöl, das reich an Linolsäure und arm an alpha-Linolensäure ist, insbesondere Passionsblumenöl oder Maisöl, oder auch ein Öl von Meerestieren, das reich an Eikosapentaen- und Dokosahexaensäuren ist, behandelt.

3. Verfahren nach Anspruch 2, das Ethylester von gamma-Linolensäure betrifft, dadurch gekennzeichnet, daß man Borretschöl behandelt.

4. Verfahren zur Herstellung eines Konzentrats von Ethylestern von mehrfach ungesättigten Fettsäuren, bei dem man eine Ethanolyse eines Öls, das reich an mehrfach ungesättigten Fettsäuren ist, durchführt, dadurch gekennzeichnet, daß man eine Verdampfung des Ethanols direkt nach der Ethanolyse durchführt, gefolgt von einer Abtrennung von Glycerin, daß man eine Vor-Raffinierung des Ethanolysats mit Aktivkohle in Gegenwart eines Lösungsmittels, insbesondere von Hexan, bei Umgebungstemperatur durchführt, daß man die Aktivkohle abtrennt und das Hexan entfernt.

5. Verfahren nach Anspruch 1 oder 2, dadurch gekennzeichnet, daß man das raffinierte Konzentrat gegen Oxydation stabilisiert, indem man ein lipidlösliches Antioxidans zusetzt.

6. Konzentrat von Ethylestern von mehrfach ungesättigten Fettsäuren, erhalten nach dem Verfahren nach Anspruch 3 ausgehend von Borretschöl, dadurch gekennzeichnet, daß es mehr als 97 Gew.-% Fettsäureethylester und weniger als 0,2 % freie Fettsäuren enthält.

7. Verwendung eines Konzentrats nach Anspruch 6 in einer Lebensmittelzusammensetzung, einer kosmetischen oder einer pharmazeutischen Zusammensetzung.

## Claims

1. Process for preparing a concentrate of the ethyl esters of polyunsaturated fatty acids, characterized in that ethanolysis is performed on a previously refined oil rich in polyunsatured fatty acids, with ethanol in the presence of a catalyst so as to obtain the ethyl esters of fatty acids, that these esters are complexed with urea dissolved in ethanol so as to form an insoluble inclusion complex, that the inclusion complex is separated and that a fraction of the ethyl esters of fatty acids enriched in polyunsaturated fatty acids is collected in the liquid phase, that ethanol is partially eliminated from the liquid phase, that the concentrate is then extracted with a solvent, that the concentrate is treated in solution at room temperature with 10 to 40 % of activated carbon based on the impure concentrate, that the activated carbon is separated and hexane is eliminated by evaporation under vacuum.

2. Process according to claim 1, characterized in that a vegetable oil is treated that is rich in gammalinolenic acid selected from evening primrose oil, borage oil or an oil from the seeds of fruits of the Ribes type, in particular blackcurrant, a vegetable oil rich in linoleic acid and low in alphalinolenic acid, in particular passion fruit oil or maize oil or furthermore a marine animal oil rich in eicosapentaenoic and docosahexaenoic acid.

3. Process according to claim 2, containing the ethyl ester of gammalinolenic acid, characterized in that borage oil is treated.

4. Process for preparing a concentrate of the ethyl esters of polyunsaturated fatty acids wherein ethanolysis is performed on an oil rich in polyunsatured fatty acids, characterized in that ethanol is evaporated off just after ethanolysis, and then after the separation of glycerol, the ethanolysate is then prerefined at room temperature with activated carbon in the presence of a solvent, in particular hexane, that the activated carbon is separated and the hexane eliminated.

5. Process according to claim 1 or 2, characterized in that the refined concentrate is stabilized against oxidation by adding an oil solubilized antioxidant.

6. Concentrate of the ethyl esters of polyunsaturated fatty acids obtained by the process according to claim 3, from borage oil, characterized in that it contains, by weight, more than 97 % ethyl esters of fatty acids and less than 0.2 % of free fatty acids.

7. Use of a concentrate according to claim 6, in a nutritive, cosmetic or pharmaceutical composition.
